# EUROPEAN PATENT SPECIFICATION

(11) **EP 0 840 750 B1**
(45) Date of publication and mention of the grant of the patent: **01.03.2006**
(21) Application number: 96911119.4
(22) Date of filing: 25.04.1996
(51) Int. Cl.: C07K 14/74, C07K 16/28, A61K 38/16, C12N 5/08

(54) **HA-2 ANTIGENIC PEPTIDE**
ANTIGENISCHE HA-2 PEPTIDE
PEPTIDE ISSU DE L'ANTIGENE HA-2

(30) Priority: 25.07.1995 EP 95202039
(43) Date of publication of application: 13.05.1998
(73) Proprietor: Rijksuniversiteit te Leiden, 2312 AV Leiden (NL); THE UNIVERSITY OF VIRGINIA PATENT FOUNDATION, Charlottesville, VA 22903 (US)
(72) Inventor: GOULMY, Els, A., J., M., NL-2343 BC Oegstgeest (NL); HUNT, Donald, F., Charlottesville, VA 22901 (US); ENGELHARD, Victor, H., Charlottesville, VA 22901 (US)
(74) Representative: Smulders, Theodorus A.H.J.
(86) International application number: PCT/NL1996/000183
(87) International publication number: WO 1997/005169

(56) References cited:
- CELL, vol. 65, no. 4, 17 May 1991, NA US, pages 633-640, XP002013530 P GRIEM ET AL.: "Uneven tissue distribution of minor histocompatibility proteins versus peptides is caused by MHC expression"
- CHEMICAL ABSTRACTS, vol. 120, no. 1, 3 January 1994 Columbus, Ohio, US; abstract no. 6418k, LI YIN ET AL.: "Few peptides dominate cytotoxic T lymphocyte responses to single and multiple minor histocompatibility antigens" page 734; XP002013531 & INT. IMMUNOL. , vol. 5, no. 9, 1993, pages 1003-1009,
- J CELL BIOL., vol. 129, no. 3, May 1995, NEW YORK, pages 819-830, XP002013599 H-E STÖFFLER ET AL.: "A novel mammalian myosin I from rat with an SH3 domain localizes to Con-A-inducible, F-actin-rich structures at cell-cell contacts"

## Description

This invention relates to the field of immunology, especially cellular immunology. It particularly relates to the area of transplantation of organs, tissues or cells (especially bone marrow) and possible immunological reactions caused by such transplantations.

Bone marrow transplantation (BMT) finds its clinical application in the treatment of, for instance, severe aplastic anaemia, leukaemia and immune deficiency disease.

In the early days many of these transplantations resulted in rejection of the transplant or in Graft versus Host Disease (GvHD). It is nowadays clear that these effects are largely due to the presence of major H antigens which function as a major transplantation barrier. Consequently, improved success in bone marrow transplantation was reported when matching for the HLA antigens was taken into account. Nowadays, mainly HLA-identical siblings or HLA-matched unrelated individuals are used as a source for the donor material. Still, despite the improvements in HLA-matching, as well as improvements in pretransplantation chemotherapy and/or radiotherapy and the use of potent immunosuppressive drugs as prophylaxis, as well as better antibiotics and better isolation techniques for the donor material, about 20-70% (depending on their age) of the recipients still suffer from GvHD. In GvHD immunocompetent donor T cells react against the host tissues. Therefore donation of marrow from which the mature T cells have been removed has become a frequently used regimen. However, this often leads to graft rejection or failure, as well as to recurrence of the original disease, which is particularly dramatic in leukaemia.

The problems still associated with (particularly) human transplantation can hardly be attributed to the major H antigens, since the donor and recipient are routinely screened for HLA identity. Therefor GvHD may be caused by the disparity of the products of the so called 'minor' H systems (mHag), i.e. Histocompatibility antigens other than those encoded by the MHC.

mHag's have been originally discovered in tumour- and skin rejection studies between congeneic strains of mice. Over 40 mHag loci have been defined, dispersed over the genome, but estimations predict the existence of several hundred loci. One of the better known minor H antigens is the HY antigen.

Several reports have demonstrated the presence of anti-host mHag specific CTL in patients suffering from GvHD after HLA genotypically identical BMT (1-7). In our laboratory, much effort was put into the further characterization of a (small) number of anti-host mHag specific CTLs. Hereto, CTL clones specific for host mHag were isolated from the peripheral blood (PBL) of patients suffering from severe GvHD (8).

Subsequent immunogenetic analyses revealed that the CTL clones (as described above) identified five non-sexlinked mHag, designated HA-1, -2, -3, -4, -5, which are recognized in classical MHC restricted fashion (8). mHag HA-3 is recognized in the presence of HLA-A1 and mHag HA-1, -2, -4 and -5 require the presence of HLA-A2. Segregation studies demonstrated that each of mHag HA-1 to HA-5 is the product of a single gene segregating in a Mendelian fashion and that HA-1 and HA-2 are not coded within the HLA region (9). The mHag differ from each other in phenotype frequencies; mHag HA-2 appeared very frequent (i.e. 95%) in the HLA-A2 positive healthy population (10).

With regard to the mHag expressed on different tissues, we observed ubiquitous versus restricted tissue distribution of the mHag analysed (11). The expression of the mHag HA-2 is restricted to the cells of the haematopoietic cell lineage, such as thymocytes, peripheral blood lymphocytes, B cells and/or monocytes. Also the bone marrow derived professional antigen presenting cells; the dendritic cells and the epidermal Langerhans cells express the mHag HA-2 (11, 12). The mHag HA-2 is also expressed on haematopoietic stem cells (13), on clonogenic leukemic precursor cells (14), as well as on freshly isolated myeloid and lymphoid leukemic cells (15).

To substantiate the importance of the human mH antigenic systems, we investigated whether the mHag are conserved in evolution between human and non human primates. Therefor, cells from non human primates were transfected with the human HLA-A2.1 gene. Subsequent analyses with our human allo HLA-A2.1 and four mHag HLA-2.1 restricted CTL clones revealed the presentation of ape and monkey allo and mHag HY, HA-1 and HA-2 peptides in the context of the transfected human HLA-A2.1 molecule by ape and monkey target cells. Furthermore, peptides were eluted from HLA-A2.1 molecules expressed on the transfected ape cells. An HA-2 positive fraction was identified that showed the same behaviour on reverse phase HPLC as the HA-2 fraction derived from human EBV-LCL. This implicates that the HA-2 peptide is conserved for at least 35 million years (16).

A prospective study was performed in order to document the effect of mHag in HLA genotypically identical BMT on the occurrence of acute (grade ≥ 2) GvHD. The results of the mHag typing using the CTL clones specific for five well defined mHag KA-1 to HA-5 demonstrated a significant correlation between mHag HA-1, -2, -4 and -5 mismatch and GvHD (17).

We aimed at the biochemical characterisation of human mH antigens. Thereto, we made use of the immunopurification and biochemical techniques succesfully applied by Rammensee and his colleagues (18, 19) to extract murine mH peptides from MHC molecules. Indeed, HPLC separation of low Mr molecules (< kD) obtained from acid treated MHC class 1 HLA-A2.1 molecules appeared successful. Fractions with sensitizing activity for the non-sexlinked mh antigen HA-2 specific CTL clones were isolated (20). To analyse the peptidic nature of the mHag HA-2, two sets of experiments were carried out. First, the sensitizing activity of the mHag containing fractions, obtained as described above, is susceptible to protease treatment; i.e. incubation of these mHag containing HPLC fractions with pronase or proteinase K abolished the sensitizing activity (21). Second, the MHC encoded TAP1 and TAP2 gene products are required for mHag peptide presentation on the cell surface. The transporter genes TAP1 and TAP2 associated with antigen presentation are required for delivery of peptides from the cytosol with the endoplasmic reticulum (22). The availability of a human celline "T2") lacking both transport and proteasome subunit genes enabled us to study the processing and presentation of human mH antigens. We demonstrated that the (rat) transport gene products TAP1 and TAP2 were required for processing and presentation of antigenic peptides from influenza virus and from the intracellular mH protein HA-2 (23).

However, until the present invention no one has succeeded in identifying amino acid sequences of antigenic peptides relevant in the mHag system,nor has anyone succeeded in the identification of the proteins from which they are derived. We have now for the first time identified a peptide which is a relevant part of mHag HA-2.

Thus this invention provides a (poly)peptide comprising a T-cell epitope obtainable from the minor Histocompatibility antigen HA-2 comprising the sequence according to claim 1.

The way these sequences are obtained is described in the experimental part. An important part of this novel method of arriving at said sequences is the purification and the choice of the starting material. Said novel method is therefor also part of the scope of this invention. However, now that the sequence is known, it is of course no longer necessary to follow that method, because the peptides can easily be made synthetically, as is well known in the art. Since routine techniques are available for producing synthetic peptides, it is also within the skill of the art to arrive at analogs or derivatives of the explicitly described peptides, which analogs and/or derivatives may have the same or at least similar properties and or activity. On the other hand analogs which counteract the activity of the explicitly described peptides are also within the skill of the art, given the teaching of the present invention.
A preferred embodiment of the present invention is the peptide with the sequence YIGEVLVSV which induces lysis of the cell presenting it at a very low concentration of peptide present. This does not imply that peptides inducing lysis at higher concentrations are not suitable. This will for a large part depend on the application and on other properties of the peptides, which were not all testable within the scope of the present invention.
The peptides and other molecules according to the invention find their utility in that they may be used to induce tolerance of the donor immune system in HA-2 negative donors, so that residual peripheral blood lymphocytes in the eventually transplanted organ or the bone marrow, as it may be does not respond to host HA-2 material in a HA-2 positive recipient. In this way GvHD may be prevented. On the other hand tolerance may be induced in HA-2 negative recipients in basically the same way, so that upon receipt of an organ or bone marrow from an HA-2 positive donor no rejection on the basis of the HA-2 material occurs. It may be the case that the HA-2 peptide acts in a non-allelic restricted manner. In that case the tolerance induction is not restricted to HA-2 negative individuals.

For tolerance induction very small doses can be given repeatedly, for instance intravenously, but other routes of administration may very well be suitable too. Another possibility is the repeated oral administration of high doses of the peptides. The peptides may be given alone, or in combination with othe peptides, or as part of larger molecules, or coupled to carrier materials in any suitable excipients.

Further applications of the peptide or derivatives thereof lie in the prophylactic administration of such to transplanted individuals to prevent GvHD. This can be done with either agonists, possibly in combination with an adjuvant, or with antagonists which may block the responsible cells. This can be done with or without the concomittant administration of cytokines.
Furthermore the peptides according to the invention can be used to prepare therapeutic agents capable of eliminating a subset of cells, directly or indirectly, especially cells of hematopoietic origin. This can be illustrated by the following examples, which refer to leukemia related therapeutic agents.
a) A HA-2 positive recipient (in bone marrow transplantation) can be subjected to an additional pre bone marrow transplant conditioning regime. This means that an agent which specifically recognizes a peptide according to the invention (a HA-2 peptide) as presented on hematopoietic cells, which agent induces elimination of the cells presenting said peptide, is administered to the recipient before transplantation. This agent will eliminate all residual cells (leukemic cells) of hematopoietic origin. Such agent include but are not limited to T cells (preferably provided with a suicide gene) and/or antibodies coupled to toxic moieties.
b) A HA-2 negative donor for Bone marrow transplantation can be vaccinated with a peptide according to the invention, a HA-2 peptide. Upon transplantation to a HA-2 positive recipient, the donor's immune system can eliminate any residual or recurrent HA-2 peptide presenting cells in the recipient which are of course leukemic.
c) A transplanted HA-2 positive recipient, transplanted with HA-2 negative (or for that matter HA-2 positive) bone marrow and suffering from recurrent disease (relapse), i.e. HA-2 positive leukemic cells, can be treated with (again) an agent which specifically recognizes a peptide according to the invention (a HA-2 peptide) as presented on hematopoietic cells, which agent induces elimination of the cells presenting said peptide. In case of HA-2 positive bone marrow being transplanted to the HA-2 positive recipient, it is still essential (in case of recurrent disease) to eliminate all HA-2 positive cells even though this includes the transplanted material, because otherwise the HA-2 positive leukemia will kill the recipient. In the latter case the patient can be retransplanted, if necessary.

Diagnostic applications are clearly within the skill of the art. They include, but are not limited to HA-2 typing, detection of genetic aberrancies and the like.

Other therapeutical applications of the peptide include the induction of tolerance to HA-2 proteins in HA-2 related (auto)immune diseases, such as possibly in Rheumatoid arthritis. On the other hand they may be used in vaccines in HA-2 related (auto)immune diseases.

On the basis of the peptide described herein genetic probes can be produced which can be used to screen for the gene encoding the protein. On the other hand such probes may be useful in detection kits as well. On the basis of the peptide described herein anti-idiotypic B cells and/or T cells and antibodies can be produced. All these embodiments have been made possible by the present disclosure and therefor are part of the present invention.
The techniques to produce these embodiments are all within the skill of the art.
Dose ranges of peptides and antibodies and/or other molecules according to the invention to be used in the therapeutical applications as described hereinbefore are usually designed on the basis of rising dose studies in the clinic. The doses for peptides may lie between about 0.1 and 1000 µg per kg bodyweight, preferably between about 1 and 10 µg per kg bodyweight.
The invention will be described in more explanatory detail in the following experimental part.

### EXPERIMENTAL

Using mHag specific CTL clones as in vitro tools, some murine and human mHag have been isolated from MHC molecules by acid elution and were shown to be peptides presented by MHC molecules (13,14). Further characterization i.e. the exact amino acid sequence of the mHag peptides and the identification of the proteins from which these mHag originate, have sofar not been reported. Only a small number of 'non-conventional defined' murine mHag, like the H-3 encoded β2 microglobulin alleles (15) and the Hmt restricted mitochondrial encoded maternally transmitted antigen (16), have been characterized. Here we report the identification, by tandem mass spectrometry, of the HLA-A2.1 restricted mHag HA-2 epitope.

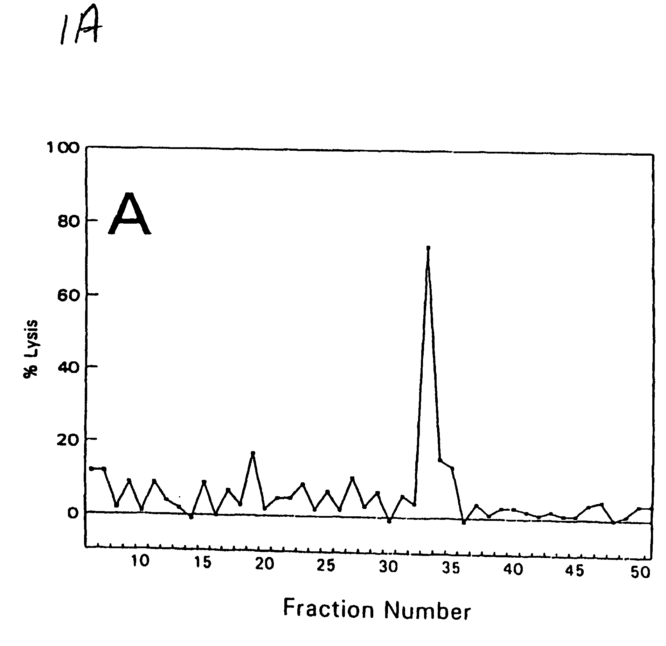

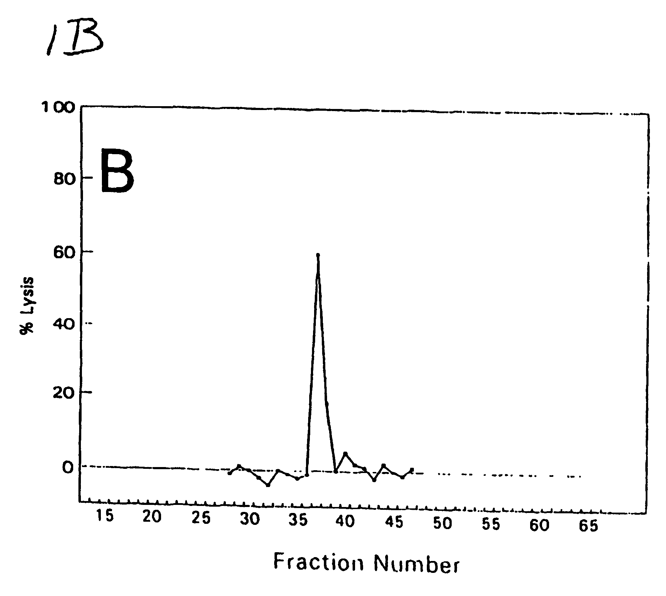

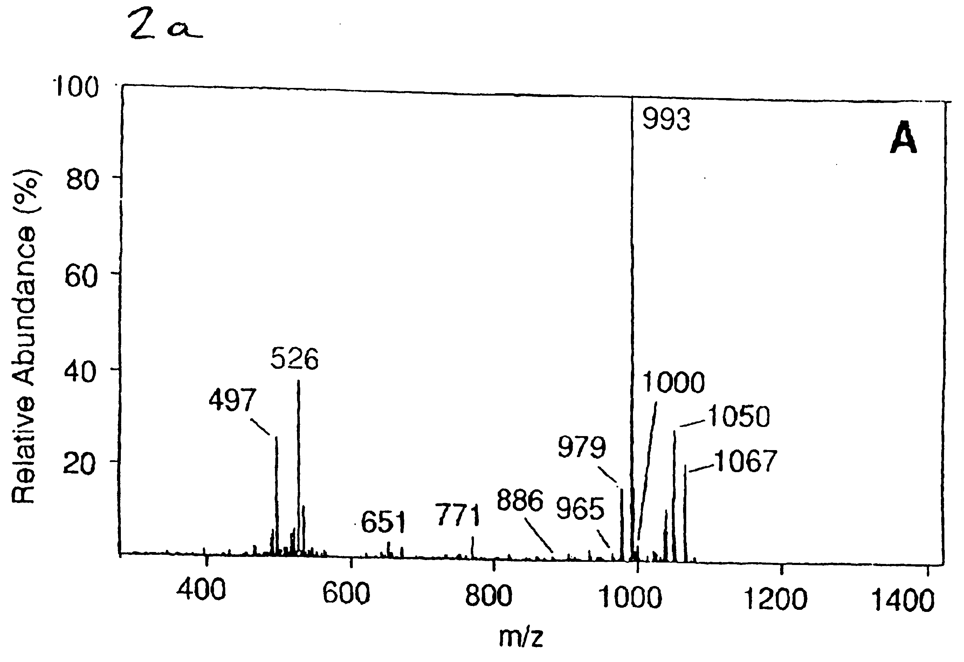

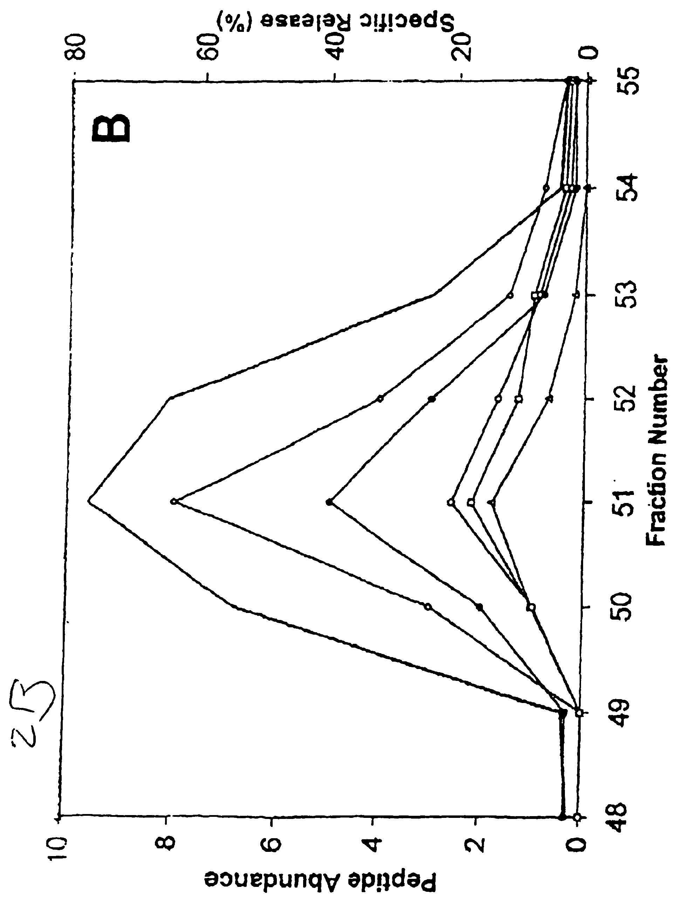

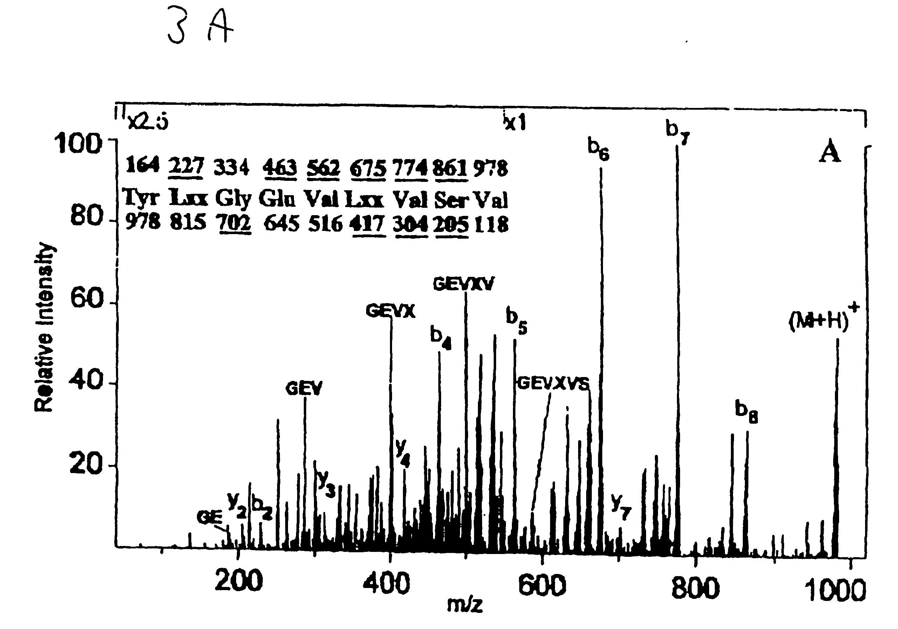

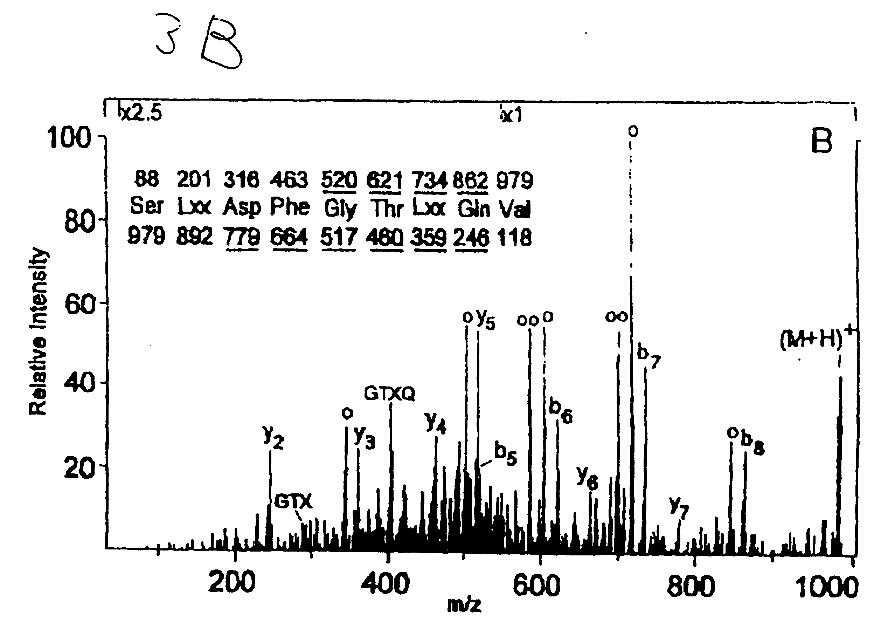

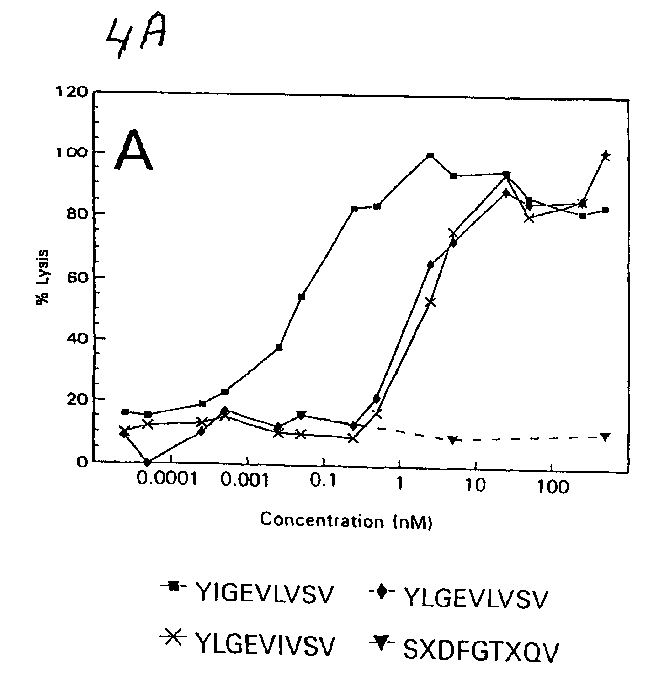

To isolate the mHag HA-2, HLA-A2.1 molecules were purified by affinity chromatography from HLA-A2.1 positive Epstein Barr Virus (EBV)-transformed B lymphocytes (EBV-BLCL) expressing HA-2. The HLA-A2.1 bound peptides were isolated by acid treatment followed by 10kD filtration (14). These low molecular mass molecules were fractionated by reverse phase HPLC and individual fractions were analyzed for mHag HA-2 sensitizing activity by incubation with the mHag HA-2 negative, HLA-A2.1 positive lymphoblastoid cell-line T2 in a ⁵¹Cr release assay. One fraction (fraction 33) sensitized T2 for lysis by the HA-2 specific CTL clone 5H17 (17) (Figure 1a). When this fraction was rechromatographed using a shallower gradient, HA-2 sensitizing activity was observed in fractions 37 and 38 (Figure 1b). However, as assessed using microcapillary HPLC/electrospray ionisation tandem mass spectrometry, the latter fractions still contained over 100 different HLA-A2 binding peptides (18). To determine which of the peptides was responsible for the HA-2 sensitizing activity, fraction 37 was analyzed using an on-line splitter (19), allowing comparison of the mass spectrometric data with results of the functional assay. Figure 2a shows a single peak of HA-2 sensitizing activity in four adjacent wells. From the many peptides present in these wells, the relative ion abundance profile of four peptides (with mass to charge ratios (m/z) of 651, 869, 979, 1000) matched the activity profile of the HA-2 specific CTL activity. Collision activated dissociation (CAD) analysis performed for the species with m/z 979 revealed the existence of 2 different peptides, YXGEVXVSV and SXDFGTXQV (figure 3a and 3b). The X stands for L or I, which cannot be distinguished by mass spectrometry under these conditions. Synthetic peptide mixtures were made with an equimolar mixture of L and I in place of X and assayed for HA-2 specific sensitizing CTL activity. Only incubation with peptide mixture YXGEVXVSV resulted in lysis of T2 (20).

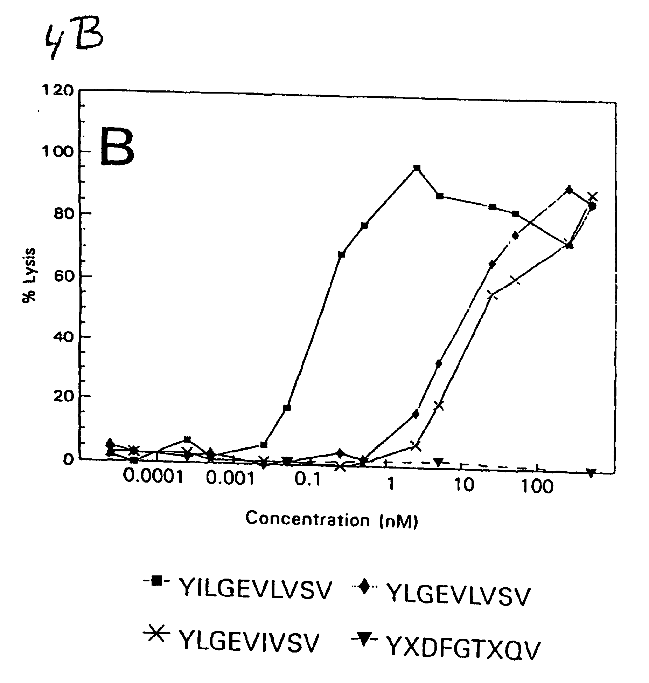

In order to further define the natural processed peptide, four single peptides were synthesized with I or L at positions two and six and microcapillary HPLC coelution studies of these synthetic peptides and the isolated fraction were performed. Peptide YIGEVIVSV did not coelute with the natural processed peptide and can therefore be excluded as the natural processed epitope, whereas the other three peptides, YIGEVLVSV, YLGEVLVSV and YLGEVIVSV did coelute (21). These three peptides all sensitized the T2 cell line for lysis by clone 5H17 (Figure 4a). Peptide YIGEVLVSV induced 50% lysis at a concentration of 40 pM, whereas these concentrations were substantially higher for peptides YLGEVLVSV and YLGEVIVSV (1.5 nM and 2.25 nM). All three concentrations are within the range of 10 pM-50 nM established for other naturally processed epitopes (19,22). Clone SH13 is an independently derived CTL that, based on panel analysis, also recognizes HA-2, but differs slightly in its fine specificity of antigen recognition from 5H17 (10,23). Clone 5H13 also recognized all 3 peptide variants (Figure 4b). While the concentration of peptides necessary to give half-maximal epitope reconstitution were 5-10 fold higher than for 5H17, peptide YIGEVLVSV still sensitized at 100 fold lower concentrations than the other two. These results establish that, despite their fine specificity differences (10,23), both HA-2 specific CTL recognize the same peptide epitope.

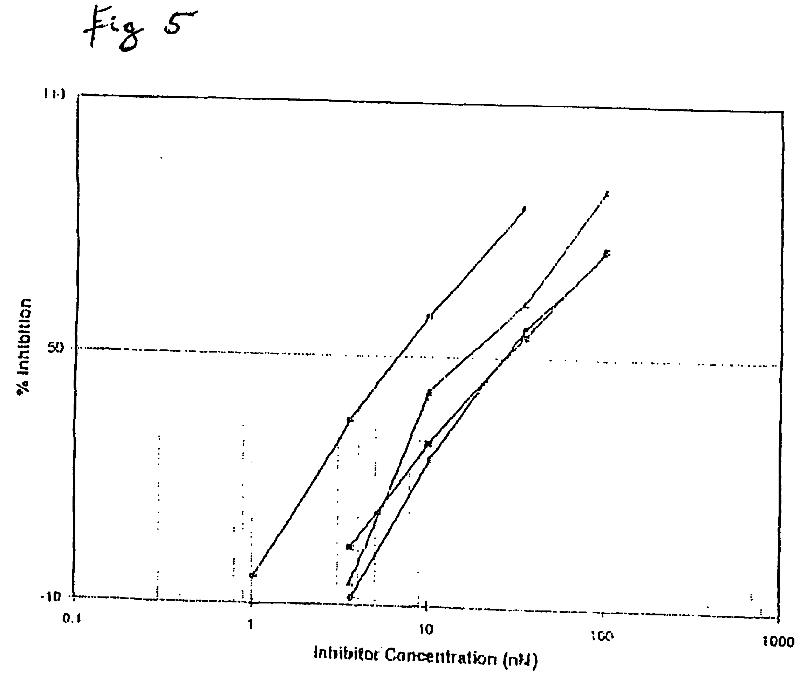

Binding studies with these three peptides showed that peptide YIGEVLVSV was the highest binder to HLA-A2.1. The concentration that resulted in 50% inhibition of the binding of the iodinated standard peptide to purified HLA-A2. 1 was 5.6 nM, while those for YLGEVIVSV and YLGEVLVSV were 9.5 and 15 nM respectively (Figure 5). These values place these peptides among the highest affinity naturally processed peptides that have been defined so far (24). However, the differences in binding affinities for these three peptides is merely a factor of 3. The fact that YIGEVLVSV sensitizes for recognition by clones 5H17 and 5H13 at 50-100 fold lower concentrations than the other two peptides indicates that this peptide is recognized with highest affinity by the TCR and thus may be the actual HA-2 epitope.

A search of DNA and protein sequence databases led to two human sequences that both matched at 7 out of 9 residues to peptide YIGEVLVSV. Peptide YYGEVCVSV is derived from oligodendrocyte myelin glycoprotein (25) and peptide YIGSVLISV was from unconventional myosin IC (26). Both human peptides were synthesized and tested for sensitizing activity. Only the myosin IC derived peptide YIGSVLISV could sensitize T2 cells for lysis by 5H17 and 5H13 with a 50% lysis inducing concentration of 5-50 nM (27). Human unconvential myosin IC belongs to a large family of myosin genes (28,29), that consist of different classes and that are indicated to be involved in cell locomotion and organelle transport (28,29). All cell types probably express several myosins from each class simultaneously. Tissue restricted distribution of some myosins has been reported (26,29). Database searches showed that in different class I myosins of various origin, ranging from Acanthamoeba castellanii to human, this peptide sequence showed conservation for Y, I, G, V, and V at position 1, 2, 3, 5 and 9. Notably, the HA-2 peptide sequence differs in the nonconserved amino acid positions from the myosin IC peptide sequence. Human unconventional myosin IC is the only cloned human class I myosin gene, but there is evidence for the presence of at least 2 other class I myosins in human cells. Therefore, it is not unlikely that an as yet unknown class I myosin protein containing YIGEVLVSV is present in humans. Interestingly, ongoing studies demonstrate the evolutionary conservation of several mHag, including HA-2, between human and non-human primates (30). Because mHag HA-2 is only presented by haematopoietic cells, this unknown class I myosin is either restricted to haematopoietic cells or is only presented by haematopoietic cells because of tissue specific processing.

The polymorphism of mHag HA-2 is an intriguing issue. 95% of the HLA-A2.1 positive population expresses the HA-2. Consequently, the HA-2 specific CTL were generated in vivo between a mHag HA-2 disparate bone marrow donor/recipient combination. The HA-2 polymorphism can be explained by either mutations in or adjacent to the HA-2 gene or by polymorphism of the antigen processing system.
Until now, information on mHag has been extremely scarce. Although the physiological function of mHag is still unknown, their pivotal role in organ transplantation in general, and in bone marrow transplantation in particular, is undeniable. We herewith report, to our knowledge for the first time, the amino acid sequence of a mHag defined by GvHD-derived CTL. The availability of the mHag peptide sequence may allow in vivo modification of the GvHD related T cell responses.
Furthermore, since mHag HA-2 is expressed on cells of the hematopoietic lineage including leukemic cells, it is a candidate for immunotherapy of leukemia prior to bone marrow transplantation.
Fig. 5. Binding of synthetic peptides to purified HLA-A2.1. HPLC purified peptides were assayed for the ability to inhibit the binding of iodinated hepatitis B core antigen peptide. FLPSDYFPSV, to purified HLA-A2.1 molecules as previously described (23). (closed circles), YIGEVLVSV; (closed triangles), YLGEVLVSV; (closed squares), YLGEVIVSV; (closed diamonds), the influenza M1 protein antigen GILGFVFTL. All data points are the average of at least two independent experiments.

### References

1. Goulmy E, Gratama JW, Blokland E, Zwaan FE, van Rood JJ (1983) A Minor transplantation antigen detected by MHC restricted cytotoxic T lymphocytes during graft-versus-host-disease. Nature 302: 159-161.
2. Tsoi M-S, Storb R, Dobbs S, Medill I, Thomas ED (1980). Cell mediated immunity to non-HLA antigens of the host by donor lymphocytes in patients with chronic graft-vs-host disease. J. Immunol. 125: 2258-2262.
3. Tsoi M-S, Storb R, Santos E, Thomas ED (1983) Anti-host cytotoxic cells in patients with acute graft-versus-host disease after HLA identical marrow grafting. Transplant Proc. 15: 1484-1486.
4. Irlé C, Beatty PG, Mickelson E, Thomas ED, Hansen JA (1985) Alloreactive T cell responses between HLA identical siblings. Transplantation 40: 329-333.
5. Van Els C, Bakker A, Zwinderman AH, Zwaan FE, van Rood JJ, Goulmy E (1990) Effector mechanisms in GvHD in response to minor Histocompatibility antigens. I. Absence of correlation with CTLs. Transplantation 50: 62-66.
6. Irscheck E, Hladik T, Niederwieser D et al (1992) Studies on the mechanism of tolerance or Graft-versus-Host Disease in allogeneic bone marrow recipients at the level of cytotoxic T cell precursor frequencies. Blood 79: 1622-1628.
7. Niederwieser D, Grassegger A, Auböck J, Herold M, Nachbaur D, Rosenmayr A, Gächter A, Nussbaumer W, Gaggl S, Ritter M and Huber C (1993) Correlation of minor histocompatibility antigen specific cytotoxic T lymphocytes with Graft-versus-Host Disease status and analyses of tissue distribution of their target antigens. Blood, 81: 2200-2208.
8. Goulmy E. Class-I restricted human cytotoxic T lymphocytes directed against transplantation antigens and their possible role in organ transplantation (1985). Prog. in allergy, vol. 36: 44-72.
9. Schreuder GMTH., Pool J, Blokland E, Van Els C, Bakker A, Van Rood JJ and Goulmy E. Genetic analysis of human minor Histocompatibility antigens demonstrates Mendelian segregation independent from HLA (1993). Immunogenetics 38: 98-105.
10. Van Els C, D'Amaro J, Pool J, Bakker A, van den Elsen PJ, Van Rood JJ and Goulmy E (1992) Immunogenetics of human minor Histocompatibility antigens: their polymorphism and immunodominance. Immunogenetics 35: 161-165.
11. De Bueger M, Bakker A, Van Rood JJ, Van der Woude F and Goulmy E. (1992). Tissue distribution of human minor Histocompatibility antigen. Ubiquitous versus restricted tissue distribution indicates heterogeneity among human CTLs defined non-MHC antigens. J. Immunology 1992, 149: 1788-1794.
12. Van Lochem EG, Van de Keur M, Mommaas M, de Gast G and Goulmy E. (1994). Expression of cytotoxic T cell defined minor Histocompatibility antigens on human peripheral blood dendritic cells and skin derived Langerhans cells, manuscript submitted for publication.
13. Marijt WAF, Veenhof WFJ, Goulmy E, Willemze R, Van Rood JJ and Falkenburg JHF (1993). Minor histocompatibility antigen HA-1, -2, -4 and HY specific cytotoxic T cell clones inhibit human hematopoietic progenitor cell growth by a mechanism that is dependent on direct cell-cell contact. Blood 82: 3778-3785.
14. Falkenburg F, Goselink H, van der Harst D, Van Luxemburg-Heijs SAP, Kooy-Winkelaar YMC, Faber LM, de Kroon J, Brand A, Fibbe WE, Willemze R and Goulmy E (1991). Growth inhibition of clonogenic leukemic precursor cells by minor histocompatibility antigen-specific cytotoxic T lymphocytes. J. Exp. Med. 174: 27-33.
15. Van der Harst D, Goulmy E, Falkenburg JHF et al (1994). Recognition of minor histocompatibility antigens on lymphocytic and myeloid leukemic cells by cytotoxic T-cell clones. Blood 83: 1060-1066.
16. Den Haan J, Pool J, Blokland E, Bontrop R and Goulmy E (1994). Minor Histocompatibility antigens are conversed between primates. Manuscript in preparation.
17. Goulmy E, Schipper R, Pool J (1994). Minor histocompatibility antigen mismatches influence the development of GvHD after HLA genotypically identical bone marrow transplantation. Manuscript subm. for publication.
18. Rötzschke O, Falk K, Wallny H-J, Faath S and Rammensee H-G (1990). Science 249: 283.
19. Falk K, Rötzschke O and Rammensee H-G (1990). Nature 348: 248.
20. De Bueger M, Verreck F, Blokland E, Drijfhout J-W, Amons R, Koning F and Goulmy E (1993). Isolation of a HLA-A2.1 extracted human minor histocompatibility peptide (1993). Eur. J. Immunol. 23: 614-618.
21. Den Haan JJM, Blokland E, Koning F, Drijfhout J-W and Goulmy E (1994). Structure analysis of human minor histocompatibility antigens HA-1 and HA-2. Abstract NWO retraite.
22. Powis SJ, Twonsend RM, Deverson EV et al. (1991) Nature 354: 528.
23. Momburg F, Ortiz-Navarrete V, Neefjes J, Goulmy E, v.d. Wal Y, Spits H, Powis SJ, Butcher GW, Howard JC, Walden P and Hammerling GJ (1992). The proteasome subunits encoded by the major histocompatibility complex are not essential for antigen presentation. Nature 360: 174-177.
24. H.J. Wallny and H-G Rammensee, Nature 343, 275 (1990). O. Rötzschke, K. Falk, H.J. Wallny, S. Faath, H-G. Rammensee, Science 249, 283 (1990). M. Sekimata, P. Griem, K. Egawa, H-G. Rammensee, M. Takiguchi, Int. Immunol. 4, 301 (1992). L. Franksson, M. Petersson, R. Kiessling, K. Karre, Eur.J. Immunol., 23, 2606 (1993);
25. M. De Bueger et al. Eur.J. Immunol.23, 614 (1993);
26. M.E. Kurtz, R.J. Graff, A. Adelman, D. Martin-Morgan, R.E. Click, J. Immunol. 135, 2847 (1985). H-G Rammensee, P.J. Robinson, A. Grisanti, M.J. Bevan, Nature 319, 502 (1986). B. Perarnau et al., Nature 346, 751 (1990);
27. B. Loveland, C-R. Wang, H. Yonekawa, E. Hermel, K. Fischer Lindahl, Cell, 60, 971 (1990);
28. The HA-2 specific CTL clone 5H17 originate from a female patient who underwent bone marrow transplantation for severe aplastic anaemia. The pre-transplant conditioning regime consisted of total lymphoid irradiation and cyclophosphamide. The patient was grafted with non-T-cell-depleted bone marrow from her HLA identical father. The patient suffered from severe acute GvHD grade III followed by extensive chronic GvHD. The HA-2 specific CTL clone was generated from post BMT PBL according to the protocol described earlier. E. Goulmy, in Transplant, Rev.J.Morris and N.L. Tilney, Eds. (Saunders Company 2, 29, 1988);
29. Data not shown;
30. A.L. Cox et al., Science 264, 716 (1994);
31. Peptide mixtures YSGEVXVSV and SXDFGTXQV were tested in several concentrations against clone 5H17 and clone 5H13. In addition to T2, an HA-2 negative HLA-A2.1 positive EBV-BLCL was used to present the peptide mixture;
32. Data not shown;
33. K. Udake, T.J. Tsomides, H.N. Eisen, cell 69, 989 (1992). R.A. Henderson et al., Proc. Natl. Acad. Sci., 90, 10275 (1993). O. Mandelboim et al., Nature, 369, 67 (1994), A. Uenaka et al., J. Exp. Med., 180, 1599 (1994);
34. 5H13 and 5H17 demonstrated identical patterns when analyzed against 100 healthy unrelated HLA-A2.1 positive individuals. A discriminatory reaction pattern between the clones was noted when a target cell was analyzed expressing a natural HLA-A2 variant molecule;
35. Y. Chen et al., J. Immunol., 152, 2874 (1994). J. Ruppert et al., Cell, 74, 829 (1993);
36.
37. W.M. Bement, T. Hasson, J.A. Wirth, R.E. Cheney, M.S. Mooseker, Proc. Natl. Acad. Sci. USA, 91, 6549 (1994);
38. Peptide YYGEVCVSV was tested in a concentration range of 50 nM to 0.5 pM against 5H17 as well as 5H13. No activity was found;
39. M.A. Titus, Curr. Opin. Cell Biol., 5, 77 (1993). E. Coudrier, A. Durrbach, D. Louvard, FEBS, 307, 87 (1992);
40. M. Mooseker, Curr. Biol., 3, 245 (1993);
41. J.M.M. den Haan, J. Pool, E. Blokland, R. Bontrop, E. Goulmy, manuscript in preparation.

## Claims

1. A peptide constituting a T-cell epitope obtainable from the minor Histocompatibility antigen HA-2 comprising the sequence YIGEVLVSV, YLGEVLVSV or YLGEVIVSV.

2. A peptide according to claim 1, comprising the sequence YIGEVLVSV.

3. A peptide according to claim 1, comprising the sequence YLGEVLVSV or YLGEVIVSV.

4. Vaccine comprising an epitope according to any one of claims 1-3.

5. A pharmaceutical formulation comprising an epitope according to any one of claims 1-3.

6. Peptide according to claims 1-3 for use as a medicine.

7. Use of a peptide according to claims 1-3 in the preparation of a medicament for the induction of tolerance for transplants to prevent rejection and/or Graft versus Host disease.

8. Use of a peptide according to any one of claims 1-3 in the preparation of a medicament for the elimination of a group of (neoplastic) hematopoietic cells presenting a peptide in the context of HLA class 1 according to any one of claims 1-3, whereby elimination is induced directly of indirectly by specific recognition of said peptide in said context.

9. Method for the generation of antibodies, T cell receptors, anti-idiotypic B-cells or T-cells, comprising the step of immunization of a non-human mammal with a peptide according to claim 1.

10. An isolated T-cell specifically recognizing the sequence YIGEVLVSV, YLGEVLVSV or YLGEVIVSV.

11. An isolated antibody specifically recognizing the sequence YIGEVLVSV, YLGEVLVSV or YLGEVIVSV.

## Patentansprüche

1. Peptid, das ein T-Zellepitop bildet erhältlich aus dem Nebenhistokompatibilitätsantigen HA-2, umfassend die Sequenz YIGEVLVSV, YLGEVLVSV oder YLGEVIVSV.

2. Peptid nach Anspruch 1, umfassend die Sequenz VIGEVLVSV.

3. Peptid nach Anspruch 1, umfassend die Sequenz YLGEVLVSV oder YLGEVIVSV.

4. Impfstoff, umfassend ein Epitop nach einem der Ansprüche 1-3.

5. Arzneimittel, umfassend ein Epitop nach einem der Ansprüche 1-3.

6. Peptid nach den Ansprüchen 1-3 zur Verwendung als Medizin.

7. Verwendung eines Peptids nach den Ansprüchen 1-3 bei der Herstellung eines Medikaments zur Toleranzinduktion für Transplantate zur Verhinderung von Abstoßung und/oder Transplantat-gegen-Wirt-Krankheit.

8. Verwendung eines Peptids nach einem der Ansprüche 1-3 bei der Herstellung eines Medikaments zur Eliminierung einer Gruppe von (neoplastischen) hämatopoietischen Zellen unter Darreichung eines Peptids in Zusammenhang mit der HLA-Klasse 1 nach einem der Ansprüche 1-3, wodurch die Elimination durch spezifische Erkennung des Peptids in dem Zusammenhang direkt oder indirekt induziert wird.

9. Verfahren zur Bildung von Antikörpern, T-Zellrezeptoren, antiidiotypischen B-Zellen oder T-Zellen, umfassend den Schritt der Immunisierung eines nicht menschlichen Säugers mit einem Peptid nach Anspruch 1.

10. Isolierte T-Zelle, die spezifisch die Sequenz YIGEVLVSV, YLGEVLVSV oder YLGEVIVSV erkennt.

11. Isolierter Antikörper, der spezifisch die Sequenz YIGEVLVSV, YLGEVLVSV oder YLGEVIVSV erkennt.

## Revendications

1. Peptide constituant un épitope de cellule T susceptible d'être obtenu de l'antigène d'histocompatibilité mineur HA-2 comprenant la séquence YIGEVLVSV, YLGEVLVSV ou YLGEVIVSV.

2. Peptide selon la revendication 1, comprenant la séquence YIGEVLVSV.

3. Peptide selon la revendication 1, comprenant la séquence YLGEVLVSV ou YLGEVIVSV.

4. Vaccin comprenant un épitope selon l'une quelconque des revendications 1 à 3.

5. Formulation pharmaceutique comprenant un épitope selon l'une quelconque des revendications 1 à 3.

6. Peptide selon les revendications 1 à 3 pour son utilisation en tant que médicament.

7. Utilisation d'un peptide selon les revendications 1 à 3 pour la préparation d'un médicament pour induire la tolérance des transplants afin de prévenir le rejet et/ou la maladie du greffon contre l'hôte.

8. Utilisation d'un peptide selon l'une quelconque des revendications 1 à 3 pour la préparation d'un médicament pour l'élimination d'un groupe de cellules hématopoïétiques (néoplasiques) présentant un peptide dans le contexte des molécules HLA de classe 1 selon l'une quelconque des revendications 1 à 3, dont l'élimination est induite directement ou indirectement par la reconnaissance spécifique dudit peptide dans ledit contexte.

9. Méthode pour générer des anticorps, des récepteurs de cellule T ou des cellules B ou T anti-idiotypiques comprenant l'étape d'immunisation d'un mammifère non-humain avec un peptide selon la revendication 1.

10. Cellule T isolée reconnaissant spécifiquement la séquence YIGEVLVSV, YLGEVLVSV ou YLGEVIVSV.

11. Anticorps isolé reconnaissant spécifiquement la séquence YIGEVLVSV, YLGEVLVSV ou YLGEVIVSV.
